# EUROPÄISCHE PATENTSCHRIFT

(11) **EP 2 253 292 B1**
(45) Veröffentlichungstag und Bekanntmachung des Hinweises auf die Patenterteilung: **29.05.2013**
(21) Anmeldenummer: 10003170.7
(22) Anmeldetag: 24.03.2010
(51) Int. Cl.: A61F 2/52

(54) **Brustprothese**
Breast prosthesis
Prothèse mammaire

(30) Priorität: 18.05.2009 DE 202009007115 U
(43) Veröffentlichungstag der Anmeldung: 24.11.2010
(73) Patentinhaber: AMOENA Medizin-Orthopädie-Technik GmbH, 83064 Raubling (DE)
(72) Erfinder: Stelter, Nils, 83112 Frasdorf (DE)
(74) Vertreter: Laufhütte, Dieter

(56) Entgegenhaltungen:
- EP-A1- 0 392 960
- WO-A2-01/82829
- WO-A2-2005/023206
- DE-A1- 10 107 189
- US-A- 4 019 209
- US-A1- 2005 020 844

## Beschreibung

Die vorliegende Erfindung betrifft ein Verfahren zur Herstellung einer abnehmaren, externen Brustprothese, umfassend einen im Wesentlichen der Form und Festigkeit der weiblichen Brust entsprechenden Körper aus einem oder mehreren weich-elastischen Kunststoffteilen besteht, die auf der hautzugewandten Seite antimikrobielle Eigenschaften aufweist.

DE-A-10107189 offenbart eine solche Brustprothese, US-A-2005/0020844 offenbart ein Elastomer, das durch Silber eine antibakterielle Wirkung erzielt.

Abnehmbare, externe Brustprothesen wurden für Frauen entwickelt, deren Brust auf Grund eines Mammakarzinoms oder einer anderen Erkrankung der Brust im Zuge einer Mastektiomie ganz oder teilweise amputiert werden mußte. Das Ziel eines sowohl postoperativen als auch dauerhaften Tragens einer Brustprothese ist vornehmlich, das Selbstbewußtsein einer betroffenen Frau nach einer Ablatio Mammae zu stärken. Um die Einschränkungen im Lebenswandel möglichst gering zu halten werden an derartige Prothesen hohe Ansprüche hinsichtlich dem optischen Erscheinungsbild und der haptischen Wahrnehmung gestellt, so daß die Form und Festigkeit der natürlichen Brust möglichst gut wiedergegeben wird. Geringes, beziehungsweise mit der natürlichen Brust vergleichbares Gewicht, ein hoher Tragekomfort, eine gute Haftung an der Haut, geringe Hitzestauung sowie gute Verträglichkeit und Hygiene zwischen der Prothese und der betroffenen, oft durch Vernarbung sensibilisierten, Hautpartie sind ebenso wichtig, um komfortables und verrutsch- und ablösefreies Tragen über einen längeren Zeitraum, auch unter Bewegung zu garantieren.

Auf Grund dieser hohen Anforderungen und des hohen Bedarfs (Brustkrebs ist in der westlichen Welt die häufigste Krebserkrankung bei Frauen) findet in diesem Bereich viel Forschung statt und folglich sind aus dem Stand der Technik viele verschiedene Brustprothesen bekannt.

Um eine Prothese möglichst stabil auf der Haut der Trägerin zu befestigen, ist aus EP 0 392 960 A1 bekannt, Brustprothesen innerhalb eines umlaufenden, lippenförmigen Randes auf ihrer Rückseite mit einer ebenfalls umlaufenden, durch einen Absatz gebildeten Schulter zu versehen. Auf dieser Schulter sind Haftstreifen oder Haftstücke befestigt, die mit den Haftbereichen der an den Körper der Frau angeklebten Streifen zusammenwirkend die Prothese mit der Haut lösbar verbinden. Als Befestigungsmittel ist dabei beispielsweise eine Klettverbindung vorgesehen.

DE 27 01 627 A1 offenbart Brustprothesen aus in Kunststoffolien eingeschweißten, der Brustform nachgebildeten schalenförmigen Körpern aus einer additionsvernetzenden Zweikomponenten-Silikonkautschuk-Masse. Derartige Prothesen geben auf Grund ihrer elastischen Weichheit, der Reaktion des Materials auf Bewegung, der fühlbaren Konsistenz sowie des Gewichts des verwendeten Materials die Eigenschaften der natürlichen Brust sehr gut wieder. Auch DE 90 10 426 U1 schlägt Brustprothesen aus einem schalenförmigen Körper aus einem weichelastisch eingestellten, additionsvernetzenden Zweikomponenten-Silikonkautschuk, der in Kunststoffolien eingeschweißt ist, vor.

Um den Tragekomfort zu erhöhen, wurde in DE 201 01 174 U1 eine Brustprothese vorgeschlagen, welche ebenfalls aus in Kunststoffolien eingeschweißten, der Brustform nachgebildeten Körpern aus einer additionsvernetzenden Zweikomponenten-Silikonkautschuk-Masse besteht. Diese weisen jedoch zumindest auf ihrer dem Körper zugewandeten Seite teilweise ein PCM (phase conversion material) Material auf, das eine Phasenumwandlungstemperatur im Bereich der Körpertemperatur hat. Dieses PCM-Material kann sich auch in einer eigenen, dem Körper zugewandten Tasche befinden. Hierdurch wird der Tragekomfort durch Vermeidung des Wärmestaus zwischen der Prothese und der Haut der Trägerin wesentlich erhöht. Eine ebenfalls einem Hitzestau zwischen der Haut der Trägerin und der Prothese entgegenwirkende Konstruktion einer Zweikammer-Brustprothese offenbart DE 44 21 516 A1. Hier ist auf der hautzugewandten Seite ein Polsterkörper aus Schaumkunststoff oder ein Faserknäuel vorhanden.

EP 0 768 068 B1 zeigt eine weitere Zweikammer-Brustprothese. Die hautzugewandte Seite weist hier ein tixotropes, vorzugsweise pastöses Material auf, das beispielsweise aus einer Mischung aus nicht vernetztem Silikonöl und Kieselsäure besteht. Eine solche Prothese eignet sich durch die besonders gute Anpassungsfähigkeit insbesondere für Patientinnen mit einer teilamputierten Brust oder einem unebenen Narbenverlauf. Derselben Problemstellung begegnet EP 0 778 013 A1. Hier ist das Füllmaterial der hautzugewandten, zweiten Kammer ein vernetzendes Material, das sich beim Anlegen an Unebenheiten anpaßt und diese Form im wesentlichen beibehält.

Eine sehr große Form- und Größenvielfalt kann mit Brustprothesen erzielen Prothesen gemäß der deutschen Gebrauchsmusteranmeldung 20 2008 008 907.7 erreicht werden. Eine solche, wiederum mehrteilige Prothese besteht einerseits aus einem schalenförmigen Körper aus einem weich-elastisch eingestellten Kunststoff, vorzugsweise einer additionsvernetzten Zweikomponenten-Silikonkautschuk-Masse, die in einer Kunststoffolie eingeschweißt ist, und zusätzlich aus einer sich an die erste Kammer anschließenden, mit einem Füllmaterial befüllbaren zweiten Kammer. Durch entsprechende Füllung dieser zweiten Kammer kann das Volumen und die Form der Brustprothese weitestgehend an die natürliche Brust der Trägerin angenähert werden.

Bei Brustprothesen aus dem Stand der Technik wurde jedoch bis zum jetzigen Zeitpunkt nicht berücksichtigt, dass die feuchte und warme Kontaktfläche zwischen der Prothese und der Haut einen ausgezeichneten Nährboden für die Bildung von Mikroorganismen wie Pilzen und vor allem Bakterien bildet. In der Folge kann sich vornehmlich, aber nicht ausschließlich, vernarbtes Gewebe entzünden. Allergische Reaktionen oder Geruchs- und Fleckenbildung durch Zersetzung des Schweißes sind ebenfalls unangenehme Nebeneffekte für die Trägerin. Dieser Problematik wurde im Stand der Technik bisher nicht begegnet.

Dieses Problem scheint jedoch relevant zu sein. DE 101 07 189 A1 offenbart Haftpflaster zur Befestigung von Prothesen, unter anderem Brustprothesen. Die Bestandteile des verwendeten Haftmittels sollen erfindungsgemäß perfekt von der Haut toleriert werden, insbesondere weil das Haftmittel in länger andauerndem Kontakt mit besonders sensibilisierter Haut verbleibt. Als möglicherweise hilfreiche Zusatzstoffe werden für das Haftmittel, unter vielen anderen, antibakterielle Reagenzien vorgeschlagen. Die Patentschrift US 6,786,798 B1 legt einen Büstenhalter für die unmittelbare, postoperative Verwendung nach verschiedenen Brustoperationen offen. Um das postoperative Entzündungsrisiko zu verringern enthält er antimikrobiell wirkende Fasern, beispielsweise solche mit Beimischung silberhältiger Salze.

Ziel der vorliegenden Erfindung ist es nun, ein Verfahren zur Hestellung einer Brustprothese zu entwickeln, die die günstigen Eigenschaften der Brustprothesen aus dem Stand der Technik beibehält und zusätzlich an den Kontaktstellen mit der Haut antimikrobiell wirkt. Hierdurch sollen der Tragekomfort und das Wohlbefinden der Frau weiter verbessert werden und Geruchs- und Fleckenbildung, Entzündungen der Haut, und ganz allgemein Erscheinungen, die durch Mikroorganismen an der Haut entstehen können, weitgehend unterbunden werden.

Dies wird durch eine eine abnehmbare, externe Brustprothese, umfassend einen im Wesentlichen der Form und Festigkeit der weiblichen Brust entsprechenden Körper aus einem oder mehreren weich-elastischen Kunststoffteilen, dadurch gekennzeichnet, dass die hautzugewandte Seite der Prothese durch eine antimikrobielle Beschichtung und/oder antimikrobielles Material in der Matrix antimikrobielle Eigenschaften aufweist, erreicht.

Eine Ausführungsform der Erfindung umfaßt ein Verfahren zur Hestellung einer abnehmbaren, externen Brustprothese, umfassend einen im Wesentlichen der Form und Festigkeit der weiblichen Brust entsprechenden Körper aus einem oder mehreren weich-elastischen Kunststoffteilen, **dadurch gekennzeichnet, dass** die hautzugewandte Seite der Prothese durch eine antimikrobielle Beschichtung und/oder antimikrobielles Material in der Matrix antimikrobielle Eigenschaften aufweist, und wobei die weich-elastischen Kunststoffteile aus einem in einer Kunststoffhülle eingeschweißten, weich-elastischen Kunststoff bestehen. In einer bevorzugten Ausführungsform ist dieser weich-elastische Kunststoff eine additionsvernetzende Zweikomponenten-Silikonkautschuk-Masse. Die Außen- und/oder Innenseite der Kunststoffhülle kann individuell aus geeigneten Kunststoffen bestehen, die bevorzugt längs ihres Umfangsrandes durch eine umlaufende Schweißnaht miteinander verschweißt sind. Besonders bevorzugt sind Kunststoffhüllen aus Polyurethan.

Die hautzugewandte Seite der Prothese weist durch eine antimikrobielle Beschichtung und/oder antimikrobielles Material in der Matrix antimikrobielle Eigenschaften auf.

Brustprothesen können auf ihrer körperzugewandten Seite ein PCM (phase change material) Material aufweisen, das eine Phasenumwandlungstemperatur im Bereich der Körpertemperatur aufweist **dadurch gekennzeichnet, dass** die hautzugewandte Seite der Prothese durch eine antimikrobielle Beschichtung und/oder antimikrobielles Material in der Matrix antimikrobielle Eigenschaften aufweist. Dies wirkt einem möglichen Hitzestau beim Tragen an der Haut entgegen.

Brustprothesen sind denkbar, deren hautzugewandte Seite tixotrope Eigenschaften besitzt, und eine optimale Anpassung der Prothese an das unebene Narbengewebe bzw. das unebene Gewebe nach einer Teilamputation der Brust zu erreichen, **dadurch gekennzeichnet, dass** die hautzugewandte Seite der Prothese durch eine antimikrobielle Beschichtung und/oder antimikrobielles Material in der Matrix antimikrobielle Eigenschaften aufweist. Alternativ oder zusätzlich zum tixotropen Material kann eine entsprechende Anpassung auch über vernetzbares Material erfolgen, welches nach einmaliger Anpassung seine Form im Wesentlichen beibehält.

Weitere Brustprothese können eine befüllbare Kammer besitzen, um eine individuelle Volumen- und Formanpassung zu ermöglichen, dadurch gekennzeichnet, dass die hautzugewandte Seite der Prothese durch eine antimikrobielle Beschichtung und/oder antimikrobielles Material in der Matrix antimikrobielle Eigenschaften aufweist.

Die Brustprothesen, weisen an der hautzugewandten Seite eine Folie auf, die eine antimikrobielle Beschichtung und/oder antimikrobielles Material in der Folienmatrix aufweist. Da eine solche Folie selbst nicht an der Haut haftet, wird diese Ausführungsform der Brustprothese entweder über einen Büstenhalter in Position gehalten, oder aber kann diese Ausführungsform mit einer der nachfolgenden Ausführungsform kombiniert werden.

So besitzen bevorzugte, erfindungsgemäße hergestellte Brustprothesen fest oder lösbar auf der hautzugewandten Seite einen Aufsatz. Ein bevorzugter Aufsatz besteht aus einem Textil, welches eine antimikrobielle Beschichtung und/oder antimikrobielles Material in der Textilmatrix aufweist. Dieses Textil kann in eine Folie eingeschweißt sein oder frei liegen. Das Textil besteht bevorzugt aus atmungsaktiven, gut hautverträglichen Fasern, die geeignete Eigenschaften im Bezug auf Schweiß- und Wärmeableitung besitzen. Geeignete Fasern umfassen beispielsweise Fasern aus einem thermoplastischen Material, Baumwolle und Mikrofasern aus Nylon, Elastan oder anderen Materialien. Es ist weiters denkbar, Polyamid- oder Polyesterfasern in beliebigem Verhältnis beizumischen, die an den Kunststoffkörper angeschweißt werden können. Das Textil kann bevorzugt klebend mit der Haut verbunden werden. Geeignete Haftmittel werden im nachfolgenden Absatz diskutiert. Eine bevorzugte Art, ein an die Haut klebbares Textil lösbar mit der Prothese zu verbinden ist ein Klettverschluß. Im Rahmen der Erfindung ist ebenfalls denkbar, dass eine textilhältige Prothese nicht an der Haut der Patientin angeklebt wird, sondern nur über einen Büstenhalter in Position gehalten wird.

In einer weiteren Ausführungsform weist die erfindungsgemäße hergestellte abnehmbare, externe Brustprothese an ihrer hautzugewandten Seite fest oder lösbar ein Haftmaterial auf, das eine antimikrobielle Beschichtung und/oder antimikrobielles Material in der Matrix aufweist. Dieses Haftmaterial ist bevorzugt ein Haftsilikon, jedoch sind andere geeignete hydrophobe Haftmittel denkbar. Solche Haftmittel umfassen unter anderem wie zwei oder mehrblockige Elastomere, z.B. vom Typ A-B oder A-B-A. Geeignete Elastomere umfassen solche mit Polystyrol- (S), Polyethen- (E), Polypropen- (P) und Polybuten- (B) Blöcken, beispielsweise in der Anordnung SEBS oder SEPS. Die Verwendung von Polyurethan und Acrylaten parallel zu EP 1 410 772 ist denkbar. Einer solchen Mischung aus synthetischen Elastomeren, sowie einem Haftsilikon können neben antimikrobiellen Materialien auch noch Harze zur Erhöhung der Klebrigkeit bzw. Weichmacher beigemischt werden.

Eine weitere, bevorzugte Ausführungsform einer erfindungsgemäßen hergestellte Brustprothese besitzt auf der hautzugewandten Seite einen Aufsatz (z.B. ein Textil) der, oder eine Beschichtung die, biologisches Material enthält. Diese(r) kann wiederum eine antimikrobielle Beschichtung und/oder antimikrobielles Material in der Matrix aufweisen. In einer besonders bevorzugten Ausführungsform wirkt das biologische Material durch Abgabe von antimikrobiellen Substanzen selbst antimikrobiell. Ein Beispiel hierfür sind die bioaktiven Stoffe in Bambusblättern, zu deren antimikrobiell und antioxidativ wirkenden Inhaltsstoffen die Polyphenole, insbesondere die Flavonoide, zählen.

Geeignete antimikrobielle Wirkstoffe umfassen solche mit einer spezifischen Wirkung, beispielsweise Bakterizide, Fungizide, oder Antiparasitika in Frage, sowie unspezifisch antimikrobiell wirkende Substanzen oder Desinfektionsmittel, die neben bakterizider, fungizider und antiparasitischer Wirkung auch Sporen und Viren abtöten können.

Von Substanzen mit spezifischer Wirkung werden beispielsweise alle Antibiotika und Anitmyotika umfasst, welche sich zum Einmischen in eine Beschichtung bzw. in eine Matrix eignen. Denkbar ist ein Einmischen von Antibiotika aus der Klasse der β-Lactame (optional gemeinsam mit β-Lactamase-Inhibitoren). Bevorzugt ist jedoch der Einsatz von Desinfektionsmitteln, die unspezifisch gegen alle Arten von Mikroorganismen, wie Bakterien, Sporen, Pilzen, Parasiten und Viren wirken. Unter den Desinfektionsmitteln eignen sich solche, welche dieser speziellen Anwendung entsprechende, besondere Anforderungen im Hinblick auf die Hautverträglichkeit erfüllen.

In einer besonders bevorzugten Ausführungsform ist in der vorliegenden Erfindung die Verwendung von Silber als antimikrobielle Substanz vorgesehen. Das Silber kann in Form von Ionen auf die Oberfläche aufgebracht oder in eine Matrix eingemischt werden, oder in feinstverteilter metallischer Form vorliegen. Es wird als kollodiales Silber, als Mikrosilber, als Nanosilber, als Silberfäden oder als Silberbeschichtung in die Matrix / das Material eines Textils, eines Haftsilikons, einer Kunststoffschicht, eines biologischen Materials oder einer Folie eingearbeitet oder als Beschichtung aufgebracht.

Mögliche Beschichtungsverfahren, wie sie von der vorliegenden Erfindung zur Aufbringung der antimikrobiellen Substanzen an die der Haut zugewandten Seite der Prothese verwendet werden, umfassen chemische und physikalische Gasphasenabscheidung, mechanische Aufbringung, Spritzen und thermisches Spritzen, Tauchlackieren, Besprühen, Aufdampfen, oder auch im Falle von elementarem Silber auch Galvanisieren. Die Einbindung des antimikrobiellen Materials in die Folienmatrix erfolgt üblicherweise schon in dessen Produktionsphase, wobei das Silber in einer Gießfolie durch Einmischen und Homogenisieren in die Lösung eingebracht werden kann und eine Extrusionsfolie über ein Masterbatch bzw. Zudosierung im Extruder entstehen kann.

Die Einbindung des antimikrobiellen Materials in die Stoffmatrix erfolgt üblicherweise schon in dessen Produktionsphase.

## Patentansprüche

1. Verfahren zur Herstellung einer abnehmbaren, externen Brustprothese, wobei die Brustprothese einen im Wesentlichen der Form und Festigkeit der weiblichen Brust entsprechenden Körper aus einem oder mehreren weich-elastischen Kunststoffteilen umfasst, und wobei die hautzugewandte Seite der Prothese eine Gießfolie mit metallischem Silber in der Folienmatrix aufweist, wodurch sie antimikrobielle Eigenschaften aufweist,
**dadurch gekennzeichnet,**
**dass** die Einbindung des Silbers in die Folienmatrix der Gießfolie in der Produktionsphase derart erfolgt, dass das Silber durch Einmischen und Homogenisieren in eine Lösung eingebracht wird.

2. Verfahren gemäß Anspruch 1, wobei die weich-elastischen Kunststoffteile aus einer additionsvernetzenden Zweikomponenten-Silikonkautschuk-Masse bestehen, die in eine Kunststoffhülle eingeschweißt sind.

3. Verfahren gemäß einem der vorhergehenden Ansprüche, wobei die hautzugewandte Seite der Prothese eine feste oder lösbare Kunststoffschicht aufweist, die metallisches Silber in der Kunststoffmatrix aufweist.

4. Verfahren gemäß einem der vorhergehenden Ansprüche, wobei die hautzugewandte Seite fest oder lösbar mit einem Haftmaterial und/oder einem Aufsatz ausgestattet ist.

5. Verfahren gemäß Anspruch 4, wobei der Aufsatz ein Textil aufweist, das metallisches Silber in der Textilmatrix aufweist.

6. Verfahren gemäß Anspruch 4 oder 5, wobei das Haftmaterial ein Haftsilikon enthält, das metallisches Silber in der Silikonmatrix aufweist.

7. Verfahren gemäß Anspruch 4 oder 5, wobei das Haftmaterial ein hydrophobes Elastomer enthält, das metallisches Silber in der Matrix aufweist.

## Claims

1. A method of manufacturing a removable, external breast prosthesis, wherein the breast prosthesis comprises a body substantially corresponding to the shape and firmness of the female breast and made from one or more flexible plastic parts, and wherein the side of the prosthesis facing the skin has a casting film with metallic silver in the film matrix whereby it has antibacterial properties,
**characterized in that**
the bonding of the silver into the film matrix of the casting film takes place in the production phase such that the silver is introduced into a solution by admixing and homogenizing.

2. A method in accordance with claim 1, wherein the flexible plastic parts comprise an addition cross-linking two-component silicone rubber compound which are welded into a plastic envelope.

3. A method in accordance with one of the preceding claims, wherein the side of the prosthesis facing the skin has a fixed or releasable plastic layer which has metallic silver in the plastic matrix.

4. A method in accordance with one of the preceding claims, wherein the side facing the skin is fixedly or releasably equipped with an adhesive material and/or with an attachment.

5. A method in accordance with claim 4, wherein the attachment has a textile which has metallic silver in the textile matrix.

6. A method in accordance with claim 4 or 5, **characterized in that** the adhesive material includes an adhesive silicone which has metallic silver the silicone matrix.

7. A method in accordance with claim 4 or 5, wherein the adhesive material includes a hydrophobic elastomer which has metallic silver in the matrix.

## Revendications

1. Procédé de fabrication d'une prothèse du sein externe amovible, la prothèse du sein comportant un corps correspondant pour l'essentiel à la forme et à la fermeté du sein de la femme, en une ou plusieurs pièces en plastique élastique souple, et la partie de la prothèse tournée vers la peau présentant une feuille mince moulée avec de l'argent métallique dans la matrice de la feuille, ce par quoi elle présente des propriétés anti-microbiennes,
**caractérisé en ce que**
l'intégration de l'argent dans la matrice de la feuille mince moulée est réalisée dans la phase de production de sorte que l'argent est introduit par immiscion et par homogénéisation dans une solution.

2. Procédé selon la revendication 1, les pièces en plastique élastique souple se composant d'une masse en caoutchouc de silicone bicomposantes réticulant additivement, qui est soudée dans une gaine en plastique.

3. Procédé selon une quelconque des revendications précédentes, le côté de la prothèse tourné vers la peau présentant une couche en plastique fixe ou décollante, qui présente de l'argent métallique dans la matrice du plastique.

4. Procédé selon une quelconque des revendications précédentes, le côté de la prothèse tourné vers la peau étant équipé de manière fixe ou décollante d'une matière adhésive et/ou d'un chapeau.

5. Procédé selon la revendication 4, le chapeau présentant un textile qui présente de l'argent métallique dans la matrice du textile.

6. Procédé selon les revendications 4 ou 5, la matière adhésive contenant une silicone adhésive qui présente de l'argent métallique dans la matrice de la silicone.

7. Procédé selon les revendications 4 ou 5, la matière adhésive contenant un élastomère hydrophobe qui présente de l'argent métallique dans la matrice.
